# EUROPEAN PATENT SPECIFICATION

(11) **EP 0 503 923 B1**
(45) Date of publication and mention of the grant of the patent: **28.12.1994**
(21) Application number: 92302077.0
(22) Date of filing: 11.03.1992
(51) Int. Cl.: C07C 401/00

(54) **Synthesis of 1 alpha-hydroxy-secosterol compounds**
Synthese von 1-Alpha-hydroxy-secosterinverbindungen
Systhèse des dérivés de 1-alpha-hydroxy secosterol

(30) Priority: 11.03.1991 US 667440; 14.02.1992 US 837010
(43) Date of publication of application: 16.09.1992
(73) Proprietor: WISCONSIN ALUMNI RESEARCH FOUNDATION, Madison, WI 53705 (US)
(72) Inventor: DeLuca, Hector F., Deerfield, Wisconsin 53531 (US); Schnoes, Heinrich K., Madison, Wisconsin 53705 (US); Perlman, Kato L., Madison, Wisconsin 53711 (US)
(74) Representative: Ellis-Jones, Patrick George Armine

(56) References cited:
- EP-A- 0 078 704
- EP-A- 0 154 185
- EP-A- 0 402 982
- WO-A-86/06255
- WO-A-91/00271
- WO-A-92/03414
- JOURNAL OF ORGANIC CHEMISTRY. vol. 51, no. 25, 12 December 1986, EASTON US pages 4819 - 4828; D. R. ANDREWS ET AL: "Synthesis of 25-Hydroxy and 1-alpha,25-Dihydroxyvitamin D3 from Vitamin D2 (Calciferol)"

## Description

This invention relates to secosterol compounds, an in particular, 1α-hydroxy-homopregnacalciferol which is a secosterol compound that is effective in inducing the differentiation of malignant cells, and finds use for the treatment of malignancies such as leukemia. More specifically, this invention relates to a novel preparation of secosterol compounds, and in particular to the preparation of 1α-hydroxy-homopregnacalciferol.

### Background of the Invention

A useful therapeutic method for the treatment of malignancies is the administration of compounds that stimulate the differentiation of malignant cells to normal cells, thereby inhibiting and/or reversing the malignant transformation. Thus, it has been shown by Suda et al (U. S. Pat. No. 4,391,802) that 1α-hydroxyvitamin D compounds (e.g. specifically 1α,25-dihydroxyvitamin D₃ and 1α-hydroxyvitamin D₃) possess, for example, potent antileukemic activity by virtue of inducing the differentiation of malignant cells (specifically leukemia cells) to non-malignant macrophages (monocytes). Hence, these compounds are useful for the treatment of certain malignancies, and specifically for the treatment of leukemia (Suda et al., U. S. Pat. No. 4,391,802). When used for such treatment, however, these known 1α-hydroxyvitamin D compounds have the disadvantage that they are also very potent calcemic agents, i.e. they cause elevated blood calcium levels by stimulating intestinal calcium absorption and bone calcium resorption. This calcemic activity represents the well known classical function of these compounds. Furthermore, the cell differentiation activity (and, hence, anti-leukemic activity) of these compounds correlates with their calcemic activity. For example, 1,25-dihydroxyvitamin D₃, the most potent compound in inducing the differentiation of malignant cells to macrophages, is also the most potent vitamin D metabolite in stimulating calcium transport or raising serum calcium levels. For practical use as cell-differentiating agents, this potent calcemic activity is, of course, an undesired side effect, since the doses required for efficacy in differentiating malignant cells can lead to excessively high and non-physiological serum calcium levels in the treated subjects.

A novel class of secosterol compounds has also been prepared (U. S. Pat. No. 4,940,700) which exhibit high differentiation activity towards malignant cells, such as leukemia cells, but do not have the undesired side effects (potent calcemic action) of some of the known compounds mentioned above. This selectivity and specificity of action makes the secosterols useful and preferred agents for the treatment of malignancies such as leukemia.

This class of secosterols is characterized by the general structures I and II shown below: 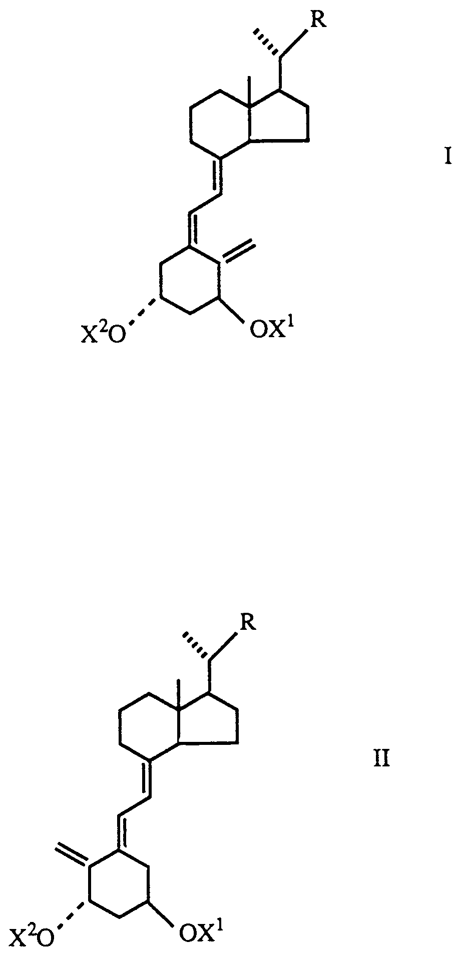
where R is methyl, ethyl or propyl and where each of X¹ and X² represent, independently, hydrogen or a hydroxy-protecting group.

Purely structurally, this class of secosterols has similarity with some of the known vitamin D compounds. Unlike the known vitamin D compounds, however, the secosterols do not express the classic vitamin D activities in vivo, i.e. stimulation of intestinal calcium transport, or the mobilization of bone calcium, and hence they cannot be classified as vitamin D derivatives from the functional point of view. These secosterols are remarkably effective in inducing the differentiation of leukemia cells to normal (non-malignant) macrophages, since, as mentioned above, potent cell differentiation activity among the known vitamin D-related compounds was always closely correlated with potent calcemic activity. Thus, the secosterols overcome the shortcomings of the known vitamin D-related anti-leukemic agents mentioned above, and can be considered preferred agents for the control and treatment of malignant diseases such as leukemia. This finding provides an effective method for the treatment of malignancies, since the above described secosterols, and preferably 1α-hydroxy-homopregnacalciferol, i.e. the secosterol of general structure I where R is methyl and X₁ and X₂ are both hydrogen, can be administered to subjects in doses sufficient to cause differentiation of malignant cells to normal cells, without producing simultaneously unphysiologically high and deleterious blood calcium levels.

A related substance, 1α-hydroxypregnacalciferol, has been prepared by Lam et al. [Steroids 26, 422 (2975)]. It should be noted that in addition to the novel synthesis disclosed herein, the secosterols of structures I and II, where R is methyl, ethyl or propyl, can also be prepared according to the general process illustrated in U. S. Pat. No. 4,940,700. Suitable starting materials for the process disclosed in 4,940,700 are i-ether steroids where, depending on the final product desired, R may be methyl, ethyl or propyl.

### Disclosure of the Invention

The present invention provides an improved and novel synthesis of secosterol compounds defined by structures I and II above. Although the specific example disclosed herein illustrates the synthesis of 1α-hydroxy-homopregnacalciferol, also known as 1α-hydroxy-20-methylpregnacalciferol, the method disclosed herein is to be considered general in nature and appropriate for synthesizing any of the secosterols defined herein. In general, the method of the present invention utilizes the reagents and conditions illustrated in Scheme I and comprises tosylating the appropriate alcohol and thereafter reducing the tosyl derivative to the desired 20-alkyl compound. The 20-alkyl compound may then be deprotected to provide the desired secosterol, namely, 1α-hydroxy-20-methyl-pregnacalciferol (commonly referred to as 1α-hydroxy-homopregnacalciferol) where R is methyl and X¹ and X² are both hydrogen, 1α-hydroxy-20-ethyl-pregnacalciferol where R is ethyl and X¹ and X² are both hydrogen, and 1α-hydroxy-20-propyl-pregnacalciferol where R is propyl and X¹ and X² are both hydrogen.

The method of making these secosterols may be broadly represented as follows wherein the method comprises the steps of providing a tosylate of the formula 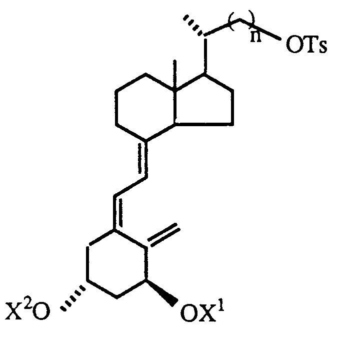
where n represents an integer being a value of 1 to 3, and X¹ and X² represent, independently, a hydroxy-protecting group, reducing the tosylate to a 20-alkyl derivative of the formula 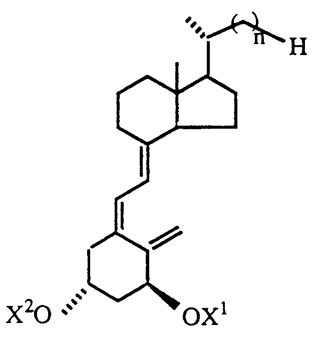
where n, X¹ and X² are as defined above, and thereafter converting the 20-alkyl derivative to the desired 1α-hydroxy-pregnacalciferol (depending upon the value of n) wherein X¹ and X² are both hydrogen.

More specifically, the present invention provides an improved and novel synthesis of 1α-hydroxy-homopregnacalciferol (synthesized earlier in U.S. Pat. No. 4,940,700), and involves a methodology similar to that provided for synthesizing side chain modified analogues of 1,25-dihydroxycholecalciferol as shown in Kutner et al, J. Org. Chem., 1988, 53, 3450. Thus, compound (6) illustrated in Process Scheme I herein, namely, (5Z,7E)-(1S, 3R, 20S)-1,3-Bis (tert-butyldimethylsilyl)-oxy-9,10-seco-22,23-dinor-5,7,10 (19)-cholatrienol 22-p-toluenesulfonate, has previously been synthesized according to that published procedure. The key step herein, however, is the reduction of the 22-tosyl derivative (6) to the 22-methyl compound (18), i.e. step XII in Process Scheme I disclosed herein. This is preferably accomplished by the nucleophilic displacement of the tosylate (6) with LiAlH₄ to give the protected homopregnacalciferol (18). Compound (18) in turn is then deprotected with tetrabutyl ammonium fluoride in THF to give compound (19), namely, 1α-hydroxy-homopregnacalciferol.

In addition to LiAlH₄, other known reducing agents may be used for the conversion of the 22-, 23-, or 24-tosyloxy intermediates to the corresponding alkyl derivatives. Suitable reagents are, for example, metal hydrides and organo metal hydrides such as the known lithium or sodium alkyaluminium hydrides, or the lithium or sodium alkoxyaluminium hydrides, dialkylaluminium hydrides, lithium trialkylborohydrides, or trialkyltin hydrides in the presence of sodium iodide.

As used herein, an acyl group is an alkanoyl group of 1 to 6 carbons in all its isomeric forms, or an aroyl group, such as benzoyl, or halo-, nitro- or alkyl -substituted benzoyl groups, or a dicarboxylic acyl group such as oxalyl, malonyl, succinoyl, glutaroyl, or adipoyl. "Alkyl" represents a straight-chain or branched saturated hydrocarbon radical of 1 to 5 carbons in all its isomeric forms, such as methyl, ethyl, propyl, isopropyl, butyl, isobutyl or pentyl. The term "hydroxy-protecting group" refers to any group used for the protection of a hydroxy function during subsequent reaction including, for example, acyl or alkylsilyl groups such as trimethylsilyl, triethylsilyl, t-butyldimethylsilyl and analogous alkylated silyl radicals, or alkoxyalkyl groups such as methoxymethyl, ethoxymethyl, methoxyethoxymethyl, tetrahydrofuranyl or tetrahydropyranyl. A "protected-hydroxy" is a hydroxy function derivatized by one of the above hydroxy-protecting groupings.

The removal of the hydroxy-protecting groups can be carried using appropriate hydrolytic or reductive procedures known in the art.

### Preparation of 1α-Hydroxy-Homopregnaciferol

### 1. 3β-Hydroxy-22,23-dinor-5,7-choladienic Acid Methyl Ester (10).

Alkaline hydrolysis of the 3β-acetoxy group of 8 followed by esterification of the carboxylic group and protection of the 3β-hydroxyl with tert-butyldimethylsilyl chloride afforded ester 9 in 72% yield. A mixture of 1.0g (2.1 mmol) of 9, 0.42g (1.5 mmol) of dibromantin, and 0.91g (10 mmol) of anhydrous sodium bicarbonate in 20mL of hexane was heated under reflux in a nitrogen atmosphere for 30 min. until no 9 was detected (TLC, hexane/ethyl acetate, 3:1). The precipitate was filtered off and the solution dried down under reduced pressure. To the solution of the residue in 5mL of anhydrous THF was added 0.06g (0.19 mmol) of tetrabutylammonium bromide, and the mixture was stirred at room temperature for 30 min. under nitrogen. A solution of tetrabutylammonium fluoride (10mL, 1M in THF) was then added followed by 0.7mL of s-collidine and the mixture was stirred under nitrogen at room temperature for 1 h. Another 5 mL of tetrabutylammonium fluoride solution was added and stirring was continued for 3 h. To the reaction mixture was added 50 mL of ether, and the organic phase was washed with water, cold 1 N hydrochloric acid, and 10% sodium bicarbonate, and dried (MgSO₄). Chromatography on 70-230-mesh silica gel (30g) with 10% ethyl acetate in hexane gave ester 10 (0.44 g 58%) as a colorless oil. An analytical sample was obtained by HPLC (system A, Rᵥ 77 mL): IR (film) 1737, 1604, 1495, 1082, 1030 cm⁻¹; UV (3% 2-propanol in hexane) λₘₐₓ 262nm (ε7000), 272nm (9800), 282 (10500), 293 (6000; ¹H NMR (CDCl₃)δ 0.54 (3H, s, 18-CH₃), 0.94 (3H, s, 19-CH₃), 1.22 (3 H, d, J=6Hz, 21-CH₃), 3.6 (1 H, m, 3-H), 3.68 (3H, s, CO₂CH₃), 5.42 (1 H, m, 6-H), 5.58 (1 H, m, 7-H); MS, m/z (relative intensity) 358 (61), 340 (12), 325 (100), 299 (68), 271 (7), 253 (17), 237 (26), 211 (27), 143 (72), 119 (35).

### 2. (5Z,7E)-(3S,20S)-3-Hydroxy-9,10-seco-5,7,10(19)-pregnatriene-20-carboxylic Acid Methyl ester (11).

A solution of 830 mg (2.3 mmol) of diene 10 in 350 mL of 1:4 (v/v) benzene-ethyl ether was irradiated with stirring under nitrogen in a water-cooled quartz immersion well equipped with a Vycor filter using Hanovia 608A36 medium-pressure UV lamp for 40 min (4 x 10 min). The reaction was monitored by HPLC using 2% 2-propariol in hexane at 25 nm. The solution was dried down under reduced pressure, redissolved in 100 mL of absolute ethanol, and heated under reflux in a nitrogen atmosphere for 3 h. Then the solution was concentrated, redissolved in 1 mL of 10% ethyl acetate in hexane, and chromatographed on 70-230-mesh silica gel (30 g). Ester 11 (298 mg, 36%) was eluted by using a mixture of 15% ethyl acetate in hexane. An analytical sample was obtained by HPLC (Zorbax silica, Phenomenex-solvent system, 2% 2-propanol in hexane): IR (film) 1738 cm⁻¹; UV (EtOH) λₘₐₓ 264 nm, λₘᵢₙ 228 nm; ¹H NMR (CDCl₃) δ 0.56 (3 H, s, 18-CH₃), 1.20 (3 H, d, J = 7 Hz, 21-CH₃), 3.66 (3 H, s, CO₂CH₃), 3.95 (1 H, m, 3-H), 4.80 (1 H, d, J = 1.2 Hz, 19Z-H), 5.05 (1 H, d, J = 1.2 Hz, 19 E-H), 6.03 (1 H, d, J = 11 Hz, 7-H), 6.23 (1 H, d, J = 11 Hz, 6-H); MS, m/z (relative intensity) 358 (M⁺, 45), 340 (9), 325 (45), 299 (22), 253 (19), 237 (18), 136 (60), 118 (100).

### 3. (7E)-(3R,5R,6R,20S)-6-Methoxy-3,5-cyclo-9,10-seco-7,10-(19)-pregnadiene-20-carboxylic Acid Methyl ester (13).

Ester 11 was converted into tosylate 12 by the known method using p-toluenesulfonyl chloride in pyridine at 4°C for 20h. A solution of 102 mg (0.2 mmol) of 12 in 2 mL of anhydrous dichloromethane was added to a solution of 250 mg of anhydrous potassium bicarbonate in 15 mL of methanol with stirring at 55°C. The mixture was stirred under nitrogen for 24 h at 55°C. The solvents were then removed under reduced pressure and the residue extracted with ether. The organic phase was washed with water and dried (MgSO₄). Silica gel chromatography, using 20% ethyl acetate in hexane, gave 13 (50 mg, 68%) as a colorless oil; ¹H NMR (CDCl₃) δ 0.54 (3H, s, 18-CH₃), 0.74 (1 H, m, 3-H), 0.91 (1 H, m, 4-H), 1.20 (3 H, d, J = 7 Hz, 21-CH₃), 3.25 (3H, s, 6R-OCH₃), 3.65 (3H, s, 22-CO₂CH₃), 4.15 (1 H, d, J = 9 Hz, 6-H), 4.88 (1 H, br s, 19Z-H), 5.00 (1 H, d, J = 9 Hz, 7-H), 5.02 (1 H, br s, 19E-H); MS, m/z (relative intensity) 372 (M⁺, 17), 340 (100), 253 (48), 221 (40), 135 (72).

### 4. (5Z, 7E)-and (5E,7E)-(1S,3R,20S)-1-Hydroxy-3-acetoxy-9,10-seco-5,7,10 (19)-pregnatriene-20-carboxylic Acid Methyl Esters (15 and 16).

tert-Butyl hydroperoxide (112µL, 3.0 M in toluene) was added to a suspension of 9 mg (0-8 mmol) of selenium dioxide in 2 µL of dry dichloromethane. The mixture was stirred at room temperature under nitrogen until a clear solution was formed. Anhydrous pyridine (12 mL, 0.15 mmol) was then added followed by a solution of 50mg of ester 13 in 2 mL of dichloromethane. The mixture was stirred under nitrogen for 30 min. Cold 10% sodium bicarbonate (2 mL) was added and the mixture extracted with ether. The organic phase was washed with cold 10% sodium bicarbonate and ice water and dried over anhydrous MgSO₄. Silica gel chromatography (10-20% ethyl acetate hexane) afforded 12.5 mg of alcohol 14. The product was then immediately dissolved in 0.5 mL of glacial acetic acid and the solution was heated at 55°C with stirring under nitrogen for 15 min. The reaction mixture was poured over ice, extracted with ether, and washed with ice-cold saturated sodium bicarbonate. The combined ether extracts were washed with water and dried (MgSO₄). Analytical samples of 5Z,7E and 5E,7E isomers, 15 and 16, respectively, were obtained by preparative HPLC in a ratio of 2.5:1. Isomers 15 and 16 were separated by the maleic anhydride procedure to give 6 mg of 15 (20% overall yield from 12).

15: HPLC, Rᵥ 68 mL; UV (EtOH) λₘₐₓ 264 nm, λₘᵢₙ 227, A264/A227 = 2.07; ¹H NMR (CDCl₃) β 0.56 (3 H, s, 18-CH₃), 1.20 (3 H, d, J = 6.5 Hz, 21-CH₃), 2.04 (3 H, s, 3β-acetyl), 3.66 (3 H, s, 22-CO₂CH₃), 4.4 (1 H, m, 1-H), 5.2 (1 H, m, 3-H), 5.01 (1 H, br s, 19E-H), 5.34 (1 H, br s, 19Z-H), 6.01 (1 H, d, J = 10 Hz, 7-H), 6.33 (1 H, d, J = 10 Hz, 6-H); MS, m/z (relative intensity), 416 (M⁺, 4), 356 (100), 338 (21), 251 (13), 134 (95).

16: HPLC, Rᵥ 78 mL; UV (EtOH) λₘₐₓ 267 nm, λₘᵢₙ 227, A267/A227 = 3.51; ¹H NMR (CDCl₃) δ 0.56 (3 H, s, 18-CH₃), 1.20 (3 H, d, J = 6.5 Hz, 21-CH₃), 2.04 (3 H, s, 3β-OAc), 3.66 (3 H, s, 22-CO₂CH₃), 4.5 (1 H, m, 1-H), 5.3 (1 H, m, 3-H), 4.99 (1 H, br s, 19E-H), 5.13 (1 H, br s, 19Z-H), 5.81 (1 H, d, J = 10 Hz, 7-H), 6.56 (1 H, d, J = 10 Hz, 6-H).

### 5. (5Z,7E)-(1S,3R,20S)-1,3-Bis[(tert-butyldimethylsily 1)-oxy]-9,10-seco-5,7,10(19)-pregnatriene-20-carboxylic Acid Methyl Ester (4).

To a stirred solution of 100 mg (0.24 mmol) of ester 15 in 10 mL of ethyl ether was added 10 mL of a 0.1 N solution of KOH in methanol. The solution was stirred at room temperature for 90 min. until no starting material was detected by TLC (hexane/ethyl acetate, 1:1). Dihydroxy ester 17 was isolated by standard extraction procedure (ethyl acetate, saturated NaCl, anhydrous MgSO₄) as a colorless oil (86.2 mg, 96%). A mixture of 250 mg (3.6 mmol) of imidazole and 250 mg (1.6 mmol) of tert-butyldimethylsilyl chloride in 2 mL of DMF was then added to a stirred solution of 86.2 mg (0.23 mmol) of 17 in 4 mL of DMF. The mixture was stirred for 15 min at 55° C until no starting material was detected by TLC (hexane/ethyl acetate, 1:1). The product was isolated with hexane. The organic extract was washed with brine and dried (MgSO₄). A hexane solution of the crude product was filtered through a silica gel SepPak cartridge to give 4 (136 mg, 98%) as a colorless oil:
IR (film) 2974, 2930, 1736, 1447, 1286, 1258, 1150, 1085 cm⁻¹; UV (hexane) λₘₐₓ 264 nm, λₘᵢₙ 227, A264/A227 =1.91; ¹H NMR (CDCl₃) δ 0.07 [12 H, s, Si(CH₃)₂]₂, 0.55 (3H, s, 18-CH₃), 0.86 [18 H, s, C(CH₃)₃], 1.20 (3 H, d, J = 6.8 Hz, 21-CH₃), 3.65 (3 H, s, OCH₃), 4.18 (1 H, m, 3-H), 4.36 (1 H, m, 1-H), 4.84 (1 H, d, J = 1.2 Hz, 19Z-H), 5.16 (1 H, d, J = 1.2 Hz, 19E-H), 5.96 (1 H, d, J = 11.2 Hz, 7-H), 6.19 (1 H, d, J = 11.2 Hz, 6-H); MS, m/z (intensities normalized to m/z 248) 602 (M⁺, 10), 470 (59), 413 (7), 338 (10), 248 (100).

### 6. (5Z,7E)-(1S,3R,20S)-1,3-Bis-(tert-butyldimethylsily 1)-oxy]-9,10-seco-22,23-dinor-5,7,10(19)-cholatrien-24-ol (5).

To a stirred solution of 136.2 mg (0.23 mmol) of ester 4 in 5 mL of anhydrous THF was added 25 mg (0.65 mmol) of lithium aluminum hydride under argon at 0°C. The suspension was stirred for 15 min at 0°C and the excess reagent was decomposed by the dropwise addition of 10% H₂O in THF. The suspension was diluted with 10 mL of THF and the stirring was continued for an additional 15 min at room temperature. The product was isolated by the standard extraction procedure with ethyl acetate. Silica gel Sep-Pak filtration in 10% ethyl acetate in hexane gave 5 (118.4 mg, 91%) as a colorless oil: IR (film) 3450, 2952, 2886, 1447, 1258, 1105, 1085, 834 cm⁻¹; UV (EtOH) λₘₐₓ 264 nm, λₘᵢₙ 227, A264/A227 = 1.57; ¹H NMR (CDCl₃) δ 0.00 (12 H, s, SiCH₃), 0.53 (3 H, s, 18-CH₃), 0.85[18H, s, SiC(CH₃)₃], 1.04(3H, d, J=6.4 Hz, 21-CH₃), 3.37 and 3.63 (1 H and 1 H, each m, 22-CH₂), 4.17 (1 H, m, 3-H), 4.35 (1 H, m, 1-H), 4.84 (1 H, br s, 19Z-H), 5.16 (1 H, br s, 19E-H), 6.00 (1 H, d, J = 12.2 Hz, 7-H), 6.21 (1 H, d, J = 12.2 Hz, 6-H); MS, m/z (intensities normalized to m/z 248), 574 (M⁺, 17), 442 (67), 383 (11), 308 (17), 248 (100).

### 7. (5Z,7E)-(1S,3R,20S)-1,3-Bis[(tert-butyldimethyisily 1)-oxy]-9,10-seco-22,23-dinor-5,7,10 (19)-chlolatrienol 22-p-Toluenesulfonate (6).

An ice-cold solution of 42.7 mg (0.22 mmol) of p-toluenesulfonyl chloride in 50 µL of dry pyridine was added to a stirred solution of alcohol 5 at 0°C under nitrogen. The mixture was stirred at 5°C for 22 h and monitored by TLC. The reaction mixture was poured on ice-cold saturated aqueous NaHCO₃ and stirring was continued for another 30 min. The product was extracted with 1:1 (v/v) ethyl ether-hexane. The organic phase was washed with saturates NaCl and dried over MgSO₄. Solvents were removes under reduces pressure and pyridine was removed in a stream of nitrogen. Crude product was purified by silica gel Sep-Pak filtration (5% ethyl acetate in hexane) to give pure tosylate 6 (54 mg, 98%): IR (film) 2950, 1580, 1367, 1267, 1189, 1178, 1099, 1085, 835 cm⁻¹; UV (hexane) λₘₐₓ 263 nm, λₘᵢₙ 236; ¹H NMR (CDCl₃) δ 0.00 (12 H, s, SiCH₃), 0.43 (3 H, s, 18-CH₃), 0.81 [18 H, s, SiC(CH₃)₃], 0.93 (3 H, d, J = 6.8 Hz, 2-CH₃), 2.40 (3 H, s, ArCH₃), 3.64 and 3.91 (1 H and 1 H, each m, 22-CH₂), 4.13 (1 H, m, 3-H), 4.31 (1 H, m, 1-H), 4.79 (1 H, br s, 19Z-H), 5.13 (1 H, br s, 19E-H), 5.94 (1 H, d, J = 12.8 Hz, 7-H), 6.17 (1 H, d, J = 12.8 Hz, 6-H), 7.43 and 7.84 (2 H and 2 H, each m, ArH); MS, m/z (intensity relative to m/z 248), 728 (6), 596 (30), 556 (7), 464 (7), 424 (44), 367 (19), 292 (23), 248 (100); exact mass calcd for C₄₁H₆₈O₅Si₂S 728.4338, found 728.4326.

### 8. 1α-hydroxy-homopregnacalciferol (19).

The tosylate 6 (7 mg, 0.1 mmole) was dissolved in 3 mL of anhydrous ether and a large excess of LiAlH₄ added by cooling. The mixture was stirred under nitrogen for 16 hours at room temperature. The mixture was cooled to 0°C and the excess LiAlH₄ carefully decomposed by the addition of wet ether. Additional ether was then added and the organic phase was washed with ice water and brine, dried and the ether removed. The residue was dissolved in ethyl acetate hexane mixture 1:1 and filtered through silica Sep-Pak and the solvents removed. The protected diol 18 was dissolved in anhydrous THF, and to this solution was added tetrabutylammonium fluoride in THF (0.5 mL, 1 M solution). The mixture was stirred under argon for 50 min at 50°C. Ether was then added and the organic phase was washed with saturated NaCl, and 10% NaHCO₃ solution. Solvents were removed and the residue was dissolved in 20% 2-propanol in hexane and filtered through a silica Sep-Pak. Preparative HPLC (column 9.4 mm x 25 cm, 20% 2-propanol in hexane) gave 1-hydroxy-homopregnacalciferol (0.5 mg) identical in all respects (UV, nmr, Mass Spec) with the previously prepared compound. UV(EtOH) λₘₐₓ 264nm, λₘᵢₙ 227nm ¹H NMR (CDCl₃) δ 0.54 (3H,s, 18-CH₃), 0.85 (1H, d, J = 7Hz, 22-H) 0.93 (1H, d, J = 7.0 Hz, 21-H), 4.23 (1H, m, 3-H), 4.42 (1H, m, 1-H), 5.0 (1H, s, 19(Z)-H) 5.34 (1H, s, 19(E)-H), 6.02 (1H, d, J = 12 Hz, 7-H), 6.39 (1H, d, J = 12 Hz, 6-H); MS, m/z (relative intensity), 330(M+, 55), 312(71), 287(7), 269(7), 251(5), 189(21), 152(73), 134(100).

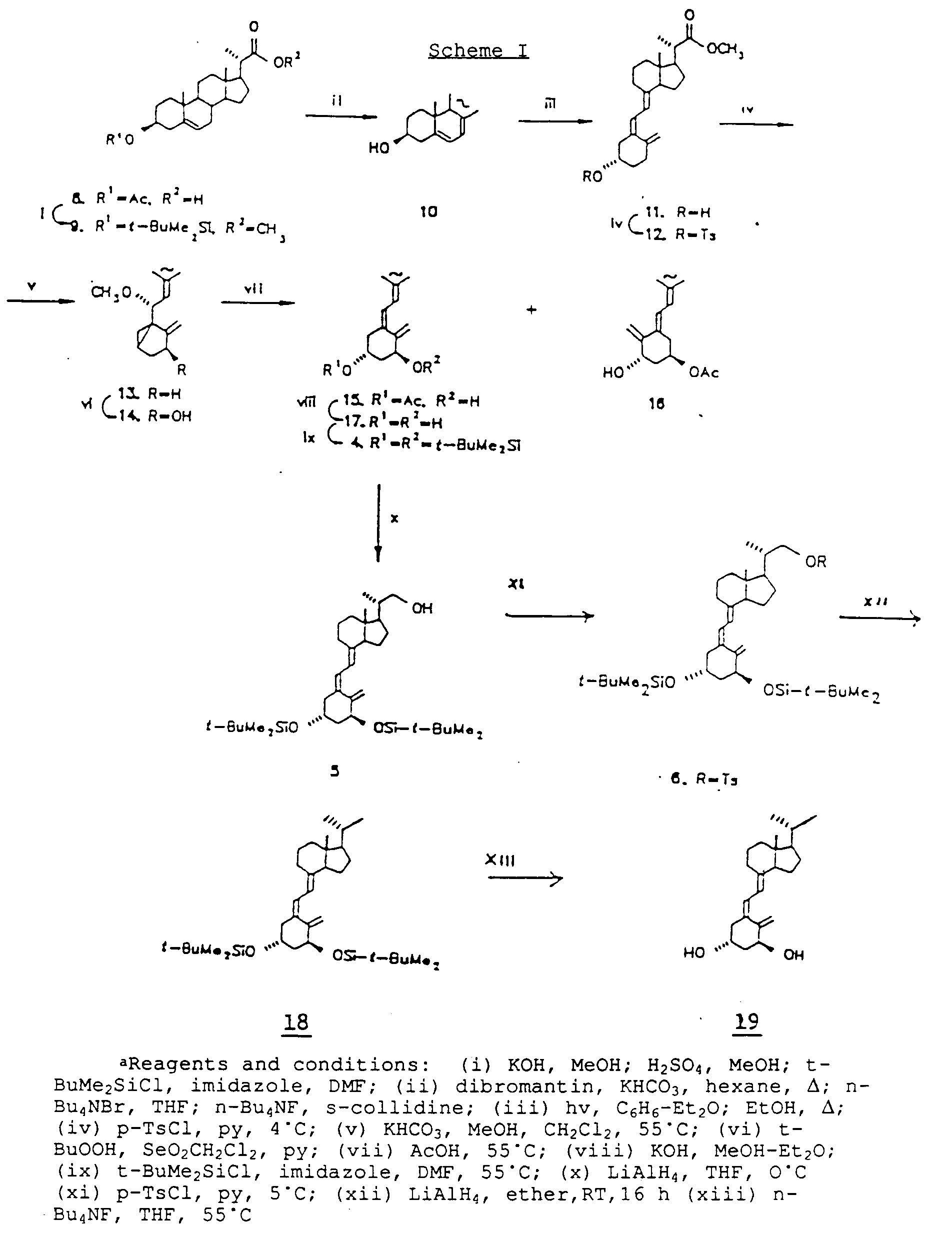

With respect to the 5,6-trans compounds of structure II, these compounds could be prepared from the compounds of structure I by known cis→trans isomerization methods, or the 5,6-trans compounds of structure II could be made by the claimed reduction method applied to the appropriate 5,6-trans-intermediates -- i.e. the further conversion of the trans compound 16 (Scheme I), using exactly the same sequence steps and conditions as shown for 15, would yield the trans analog corresponding to 19.

Whereas Scheme I only illustrates the preparation of the 22-tosylate required for the synthesis of the homopregna-compound 19, the corresponding 23-tosylate and 24-tosylate that would be needed for the synthesis of the 20-ethyl and 20-propyl pregnacalciferol compounds, respectively, can be prepared quite readily from the corresponding esters. These esters are available through processes disclosed in U. S. Patents 4,195,027, 4,260,804 and 4,267,117. Their further conversion by process steps analogous to the steps shown in Scheme I for the conversion of compound 17 to compound 6 then provides the corresponding 23- and 24-tosylates; these 1α-hydroxyvitamin-23-tosylate and 1α-hydroxyvitamin-24-tosylate derivatives are new compounds.

### Alternative Synthesis of Compounds I and II where R=Ethyl or Propyl

The compounds of structure I or II above, where R is ethyl or propyl can also be prepared by an alternative procedure involving displacement of the 22-tosyloxy group in an intermediate of structure 6 (or of the corresponding 5,6-trans derivative) by an alkyl group. Thus, treatment of compound 6-with methyl magnesium bromide (or analogous methyl Grignard reagent) affords compound I, where R=ethyl. Similarly, reaction of compound 6, with ethyl magnesium bromide yields compound I where R=propyl. In like fashion, the 5,6-trans derivatives of 6, reacted with methyl or ethyl magnesium bromide, afford compounds II, where R represents ethyl and propyl, respectively. These tosyl displacement reactions are advantageously conducted in the presence of cuprous iodide, in an organic solvent, preferably an ether solvent such as diethylether or tetrahydrofuran, at a temperature from about 0°C to the boiling temperature of the solvent, preferably at about room temperature.

## Claims

1. A method of making a 1α-hydroxy-secosterol which comprises reducing a tosylate of the formula: 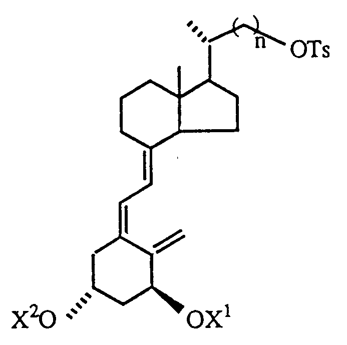 where n represents an integer having a value of 1 to 3, and X¹ and X² represent, independently, a hydroxy-protecting group, with a metal hydride or organo metal hydride reducing agent to a 20-alkyl derivative of the formula: 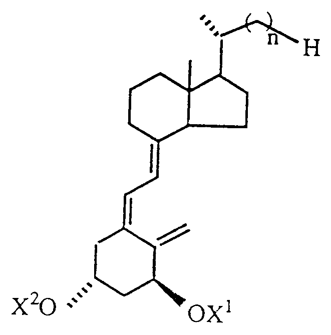 where n is as defined above and X¹ and X² represent, independently, hydrogen or a hydroxy protecting group, and when X¹ and/or X² in the 20-alkyl derivative are/is hydroxy protecting groups converting the 20-alkyl derivative to the desired 1α-hydroxy-secosterol wherein X¹ and X² are both hydrogen.

2. A process according to claim 1 wherein prior to the converting step X¹ and X² are both t-BuMe₂Si.

3. A process according to claim 1 or 2 wherein the hydroxy protecting group is an acyl group.

4. A process according to any one of claims 1 to 3 wherein the desired secosterol is 1α-hydroxy-20-methyl-pregnacalciferol, 1α-hydroxy-20-ethyl-pregnacalciferol or 1α-hydroxy-20-propyl-pregnacalciferol.

5. A process according to any one of claims 1 to 4 wherein the reducing agent is lithium aluminium hydride, a lithium alkylalumium hydride, sodium alkylalumium hydride, lithium alkoxyaluminium hydride, sodium alkoxyaluminium hydride, dialkylalumium hydride, lithium trialkylborohydride or trialkyltin hydride.

6. A process according to any one of claims 1 to 3 and 5 for making 1α-hydroxy-homopregnacalciferol where n is 1.

7. A method for preparing a 1α-hydroxysecosterol compound of the formula: 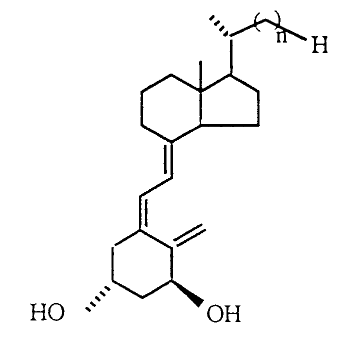 where n represents an integer having a value of 2 or 3, which comprises reacting a vitamin D 22-tosylate derivative of the formula: 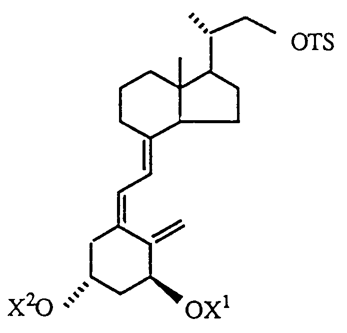 where X¹ and X² represent, independently, hydroxy-protecting groups with a methyl or ethyl magnesium halide reagent in an organic solvent to produce the hydroxysecosterol reaction product and when X¹ and/or X² in the reaction product are/is hydroxy protecting groups converting the reaction product to the desired hydroxy secosterol.

8. A process according to claim 7 wherein the secosterol compound is 1α-hydroxy-20-ethyl-pregnacalciferol, or 1α-hydroxy-20-propyl-pregnacalciferol.

9. A process according to claim 7 or 8 wherein the hydroxy-protecting group is an acyl or a tri-alkylsilyl group.

## Patentansprüche

1. eine Methode zur Herstellung eines α-Hydroxy-secosterins, die umfaßt Reduktion eines Tosylats der Formel: 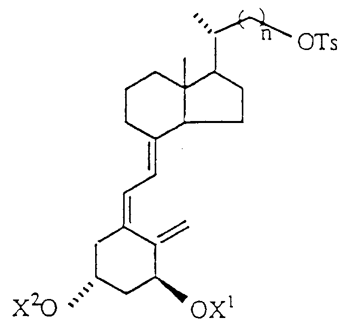 worin n eine ganze Zahl eines Werts von 1 bis 3 darstellt, und X¹ und X² unabhängig eine Hydroxyschutzgruppe darstellen, mit einem Metallhydrid oder Organometallhydrid-Reduktionsmittel zu einem 20-Alkyl-Derivat der Formel: 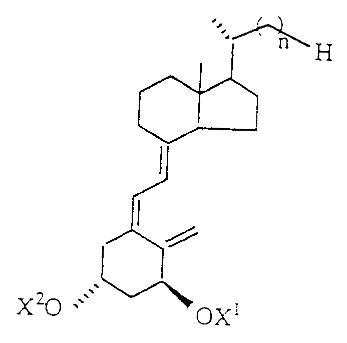 worin n definiert ist wie oben und X¹ und X² jeweils unabhängig Wasserstoff oder eine Hydroxyschutzgruppe darstellen, und wenn X¹ und/oder X² in dem 20-Alkyl-Derivat Hydroxyschutzgruppen sind/ist, Umwandlung des 20-Alkyl-Derivats zum angestrebten 1α-Hydroxy-secosterin, worin X¹ und X² jeweils Wasserstoff sind.

2. Ein Verfahren entsprechend Anspruch 1, worin vor dem Umwandlungsschritt X¹ und X² beide t-BuMe₂Si sind.

3. Ein Verfahren entsprechend Anspruch 1 oder 2, worin die Hydroxy-Schutzgruppe eine Acylgruppe ist.

4. Ein Verfahren entsprechend einem der Ansprüche 1 bis 3, worin das angestrebte Secosterin 1α-Hydroxy-20-methylpregnacalciferol, 1α-Hydroxy-20-ethyl-pregnacalciferol oder 1α-Hydroxy-20-propyl-pregnacalciferol ist.

5. Ein Verfahren entsprechend einem der Ansprüche 1 bis 4, worin das Reduktionsmittel Lithiumaluminiumhydrid, ein Lithiumalkylaluminiumhydrid, Natriumalkylaluminiumhydrid, Lithiumalkoxyaluminiumhydrid, Natriumalkoxyaluminiumhydrid, Dialkylaluminiumhydrid, Lithiumtrialkylborhydrid oder Trialkylzinnhydrid ist.

6. Ein Verfahren entsprechend einem der Ansprüche 1 bis 3 und 5 zur Herstellung von 1α-Hydroxy-homopregnacalciferol, worin n 1 ist.

7. Ein Verfahren zur Herstellung einer 1α-Hydroxysecosterin-Verbindung der Formel: 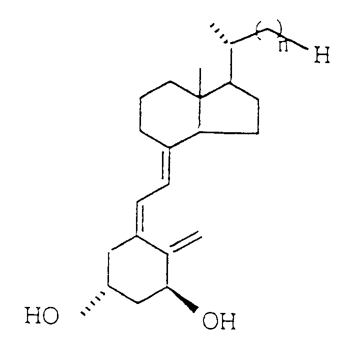 worin n eine ganze Zahl eines Werts von 2 bis 3 darstellt, das umfaßt Reaktion eines Vitamin D 22-Tosylat-Derivats der Formel 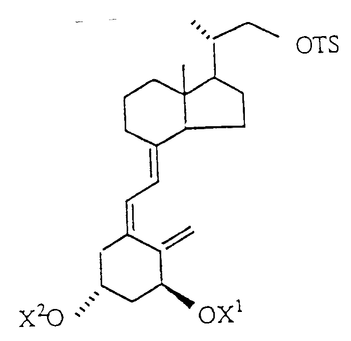 worin X¹ und X² unabhängig Hydroxy-Schutzgruppen darstellen, mit einem Methyl- oder Ethylmagnesiumhalogenid-Reagenz in einem organischen Lösungsmittel zur Herstellung des Hydroxysecosterin-Reaktionsprodukts und, wenn X¹ und/oder X² in dem Reaktionsprodukt Hydroxyschutzgruppen sind/ist, Umwandlung des Reaktionsprodukts in das angestrebte Hydroxysecosterin.

8. Ein Verfahren entsprechend Anspruch 7, worin die Secosterin-Verbindung 1α-Hydroxy-20-ethyl-pregnacalciferol oder 1α-Hydroxy-20-propyl-pregnacalciferol ist.

9. Ein Verfahren entsprechend Anspruch 7 oder 8, worin die Hydroxy-Schutzgruppe eine Acyl- oder Trialkylsilylgruppe ist.

## Revendications

1. Procédé de préparation d'un 1α-hydroxy-sécostérol, qui comporte le fait de réduire un tosylate de formule : 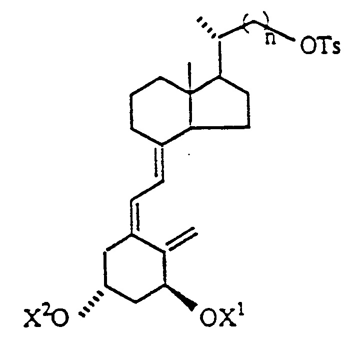 dans laquelle n représente un nombre entier valant de 1 à 3, et X¹ et X² représentent chacun, indépendamment, un groupe hydroxy-protecteur,
à l'aide d'un agent réducteur qui est un hydrure métallique ou un hydrure organométallique, en un dérivé 20-alkylé de formule : 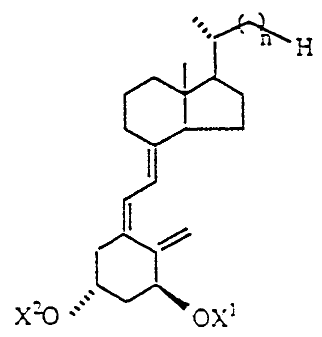 dans laquelle n est tel qu'on l'a défini ci-dessus, et X¹ et X² représentent chacun, indépendamment, un atome d'hydrogène ou un groupe hydroxy-protecteur,
et quand X¹ et/ou X² représentent, dans le dérivé 20-alkylé, un groupe hydroxy-protecteur, le fait de convertir ce dérivé 20-alkylé en le 1α-hydroxy-sécostérol voulu, où X¹ et X² représentent tous les deux un atome d'hydrogène.

2. Procédé conforme à la revendication 1, dans lequel X¹ et X² représentent tous les deux, avant l'étape de conversion, un groupe t-BuMe₂Si.

3. Procédé conforme à la revendication 1 ou 2, dans lequel le groupe hydroxy-protecteur est un groupe acyle.

4. Procédé conforme à l'une des revendications 1 à 3, dans lequel le sécostérol voulu est le 1α-hydroxy-20-méthyl-prégnacalciférol, le 1α-hydroxy-20-éthyl-prégnacalciférol ou le 1α-hydroxy-20-propyl-prégnacalciférol.

5. Procédé conforme à l'une des revendications 1 à 4, dans lequel l'agent réducteur est de l'hydrure de lithium et d'aluminium, un hydrure de lithium et d'alkyl-aluminium, un hydrure de sodium et d'alkyl-aluminium, un hydrure de lithium et d'alcoxy-aluminium, un hydrure de sodium et d'alcoxy-aluminium, un hydrure de dialkylaluminium, un trialkylborohydrure de lithium ou un hydrure de trialkyl-étain.

6. Procédé conforme à l'une des revendications 1 à 3 et 5, permettant de préparer du 1α-hydroxy-homoprégnacalciférol dans lequel n vaut 1.

7. Procédé de préparation d'un 1α-hydroxy-sécostérol de formule : 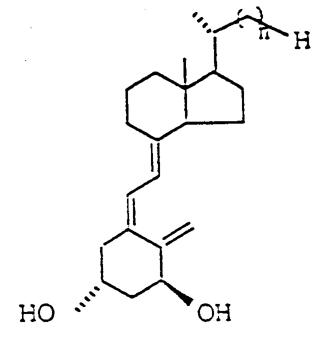 dans laquelle n représente un nombre entier valant 2 ou 3,
qui comporte le fait de faire réagir un dérivé 22-tosylate de vitamine D, de formule : 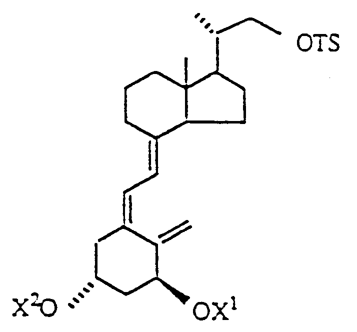 dans laquelle X¹ et X² représentent chacun, indépendamment, un groupe hydroxy-protecteur,
avec un réactif de type halogénure de méthyl-magnésium ou d'éthylmagnésium, dans un solvant organique, afin d'obtenir un produit de réaction qui est un hydroxysécostérol, et quand X¹ et/ou X² représentent, dans ce produit de réaction, un groupe hydroxy-protecteur, le fait de convertir ce produit de réaction en l'hydroxy-sécostérol voulu.

8. Procédé conforme à la revendication 7, dans lequel le sécostérol voulu est le 1α-hydroxy-20-éthyl-prégnacalciférol ou le 1α-hydroxy-20-propyl-prégnacalciférol.

9. Procédé conforme à la revendication 7 ou 8, dans lequel le groupe hydroxy-protecteur est un groupe acyle ou un groupe trialkylsilyle.
